# EUROPEAN PATENT APPLICATION

(11) **EP 4 020 462 A1**
(43) Date of publication of application: **29.06.2022**
(21) Application number: 21217554.1
(22) Date of filing: 23.12.2021
(51) Int. Cl.: G10K 15/02

(54) **SYSTEM FOR PRODUCING MUSIC FOR WELLBEING FROM A PERSONAL ARTERIAL PULSE WAVE FORM**

(30) Priority: 24.12.2020 US 202063199419 P
(71) Applicant: Lipot, Mordehai Roland, 96431 Jerusalem (IL); Lipot, Israel Meir, 96431 Jerusalem (IL)
(72) Inventor: Lipot, Mordehai Roland, 96431 Jerusalem (IL); Lipot, Israel Meir, 96431 Jerusalem (IL)
(74) Representative: Arnold & Siedsma

(57) **Abstract**

There is provided a system of producing music that is in accordance with a user's measured arterial pulse wave form, the system comprising: a sound generator comprising a processing circuitry, the processing circuitry comprising a processor and memory, the processing circuitry being configured to: receive a digital signal indicative of a plethysmogram (PG) of the user; and generate, from the digital signal, data indicative of an audio segment.

## Description

### TECHNICAL FIELD

The presently disclosed subject matter relates to music producing devices, and in particular music devices integrating user sensor input.

### BACKGROUND

Problems of producing music for personal relaxation and wellbeing have been recognized in the conventional art, and various techniques have been developed to provide solutions.

The human arterial pulse has been described as follows:
.. the result of a wave of vascular distention, initiated by the impact of the stroke volume ejected into a closed system with every heartbeat. The forward-propagating pressure wave has both a fast-moving (10m/sec) and slower (0.5m/sec) component. The wave is reflected back by the arteriole, which provides the majority of peripheral vascular resistance
Peak aortic blood flow acceleration produces the initial rise of the pressure pulse, whereas the ejection of the ventricular volume fills out and sustains the pulse waveform. The systolic components follow the electrocardiogram (ECG) R wave. The interval between the two reflects the time required for the spread of a depolarisation wave, isovolumic left ventricular contraction, aortic valve opening, left ventricular ejection, transmission of the aortic pressure wave to the radial artery and of the pressure signal from catheter to transducer. The incisura is the dicrotic notch recorded directly from the central aorta and relates to aortic valve closure. The peripheral arterial waveform has a later, smoother dicrotic notch that reflects arterial wall properties.
Simultaneous recording of pressure waveforms from different arterial sites have different morphologies because of the impedance and harmonic resonance of the vascular tree. Predominantly pressure wave reflection influences the shape of the waveform as it travels peripherally, because the high resistance to flow in the arterioles diminishes pressure pulsations in small downstream vessels but augment upstream arterial pressure pulses owing to wave reflection.

[REGISTRAR PRIZE Southern African Journal of Anaesthesia & Analgesia - February 2003]

### GENERAL DESCRIPTION

According to one aspect of the presently disclosed subject matter there is provided a system of producing music that is in accordance with a user's measured arterial pulse wave form, the system comprising a processing circuitry comprising a processor and memory, the processor being configured to:
**a.** receive a digital signal indicative of a plethysmogram (PG) of the user; and
**b.** generate, from the digital signal, data indicative of an audio segment.

In addition to the above features, the method according to this aspect of the presently disclosed subject matter can comprise one or more of features (i) to (ix) listed below, in any desired combination or permutation which is technically possible:
(i) additionally comprising a waveform detector operably connected to the sound generator and configured to provide the digital signal to the sound generator, the waveform detector comprising:
   **a.** a sensor configured to provide data indicative of a volume of blood within tissue of the user, and
   **b.** a digitizer configured to:
      generate, from the data indicative of the volume of blood, data indicative of a digital signal,
      the digital signal being indicative of a PG.
(ii) to generate the data indicative of the audio segment, the processing circuitry is further configured to:
   **a.** determine, for a given time, an amplitude of the digital signal;
   **b.** select an audio tone from a plurality of audio tones in accordance with the determined amplitude; and
   **c.** repeat a.-b. for zero or more additional times.
(iii) the processing circuitry is further configured to:
   **a.** utilize waveform analysis to determine, for a given interval of the digital signal, a pulse type;
   **b.** select a sequence of audio tones from a plurality of sequences of audio tones in accordance with the determined pulse type; and
   **c.** repeat a.-b. for zero or more additional intervals.
(iv) the processing circuitry is further configured to:
   **a.** utilize waveform analysis to determine, for a given interval of the digital signal, a pulse type;
   **b.** determine, for a given time, an amplitude of the digital signal;
   **c.** select an audio tone from a first plurality of audio tones in accordance with the determined amplitude,
      wherein the first plurality of audio tones is selected from a group of audio tones in accordance with the determined pulse type;
   **d.** repeat b.-c. for zero or more additional times; and
   e. repeat a.-d. for zero or more additional intervals.
(v) the processing circuitry is further configured to play the audio segment on a speaker.
(vi) the processing circuitry is further configured to store data indicative of the audio segment to a storage medium.
(vii) the processing circuitry is further configured to store, to a storage medium, musical score data that is in accordance with the audio segment.
(viii) the processing circuitry is further configured to generate visualization data in accordance with the digital signal.
(ix) the sensor is selected from a group consisting of:
   a pulse oximeter,
   a blood pressure sensor, and an accelerometer.

According to a further aspect of the presently disclosed subject matter there is provided a processing circuitry-based method of producing music that is in accordance with a user's measured arterial pulse wave form, the method comprising:
**a.** receiving a digital signal indicative of a plethysmogram (PG) of the user; and
**b.** generating, from the digital signal, data indicative of an audio segment.

This aspect of the disclosed subject matter can further optionally comprise one or more of features (i) to (ix) listed above with respect to the system, mutatis mutandis, in any desired combination or permutation which is technically possible.

According to another aspect of the presently disclosed subject matter there is provided a computer program product comprising a non-transitory computer readable storage medium retaining program instructions, which, when read by a processing circuitry, cause the processing circuitry to perform a method of a method of producing music that is in accordance with a user's measured arterial pulse wave form, the method comprising:
**a.** receiving a digital signal indicative of a plethysmogram (PG) of the user; and
**b.** generating, from the digital signal, data indicative of an audio segment.

This aspect of the disclosed subject matter can further optionally comprise one or more of features (i) to (ix) listed above with respect to the system, mutatis mutandis, in any desired combination or permutation which is technically possible.

### BRIEF DESCRIPTION OF THE DRAWINGS

In order to understand the invention and to see how it can be carried out in practice, embodiments will be described, by way of non-limiting examples, with reference to the accompanying drawings, in which:
**Fig. 1** illustrates examples of arterial pulse forms, in accordance with some embodiments of the presently disclosed subject matter;
**Fig. 2** illustrates a block diagram of an example music generation system, in accordance with some embodiments of the presently disclosed subject matter;
**Fig. 3** illustrates a plethysmogram together with its first and second derivatives, in accordance with some embodiments of the presently disclosed subject matter;
**Fig. 4** illustrates examples of a flow diagram of an example method of deriving sound data from a user's plethysmogram signal, in accordance with some embodiments of the presently disclosed subject matter; and
**Fig. 5** illustrates a flow diagram of an example method of utilizing waveform analysis to generate or modify the music derived from a plethysmogram signal, in accordance with some embodiments of the presently disclosed subject matter;

### DETAILED DESCRIPTION

Different people typically exhibit different arterial pulse forms, and individuals exhibit different arterial pulse forms in different circumstances.

Mystical traditions ascribe special significance to the rate and shape of the heartbeat and pulse.

Arterial pulse forms can be measured._In each cardiac cycle, the heart pumps blood to the periphery. An optical sensor can detect blood volume changes in the microvascular bed of tissue by measuring changes in light absorption.

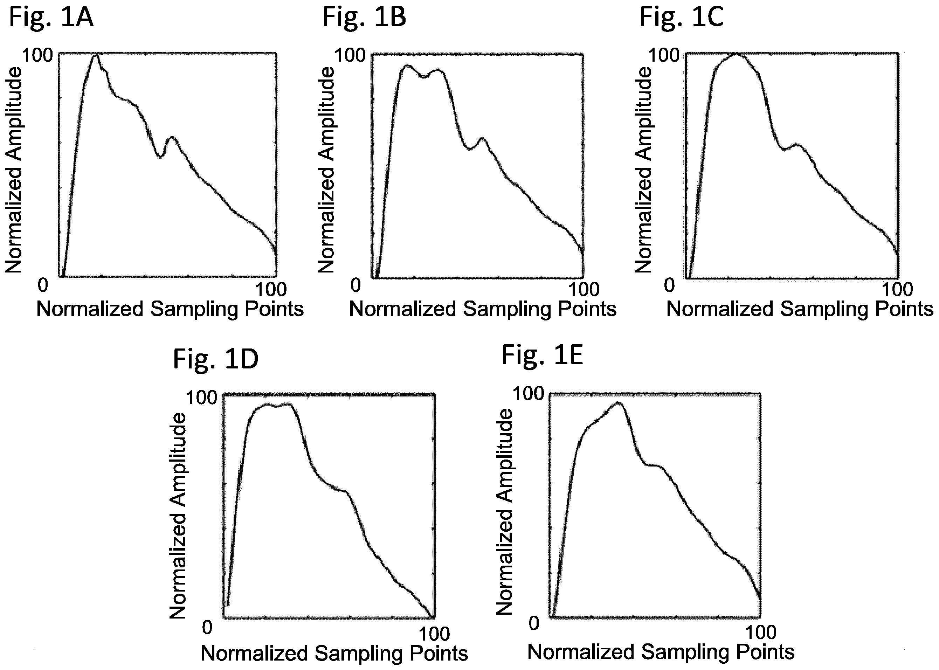

By way of non-limiting example: a pulse oximeter monitors the perfusion of blood to the dermis and subcutaneous tissue of the skin. The pulse oximeter illuminates the skin (for example: with light from a light-emitting diode (LED)), and then measuring the amount of light either transmitted or reflected to a photodiode. Even though this pressure pulse can somewhat damped by the time it reaches the skin, it can be enough to distend the arteries and arterioles in the subcutaneous tissue. Each cardiac cycle appears as a peak, as seen in **Fig. 1**. If the pulse oximeter is attached without compressing the skin, a pressure pulse can also be seen from the venous plexus, as a small secondary peak.

A photoplethysmogram (PPG) is an optically obtained plethysmogram (PG) describing e.g. measured or estimated blood volume over time.

Other sensors such as - for example - a pressure sensor or an accelerometer can be used to measure blood volume over time. Such devices can also produce data indicative of a plethysmogram.

Attention is directed to **Fig. 1****,** which illustrates examples of arterial pulse forms. The x-axis represents a succession of sampling points, while y-axis represents the blood volume.

Some embodiments for the presently disclosed subject matter perform analysis on received PPG data, and utilize the resulting signal to produce music that is derived from the arterial pulse forms.

Some practitioners report enjoyment as well as physical and spiritual benefit, relaxation, improved concentration, cognitive and memory improvement, and general wellbeing from listening to music derived in this manner.

There exist various techniques for analysis of PPG data, for example:
it is possible to further simplify the interpretation and understanding of the original PPG signal by taking its first derivative and second derivative The second derivative of the PPG signal is called the Acceleration Plethysmogram (APG). The APG is indicative of blood acceleration in the finger. The APG waveform that is extracted from the proposed model is comprised of two systolic waves and one diastolic wave. These waves are named as follows: the a-wave (known as the early systolic positive wave), the b-wave (known as the early systolic negative wave) and the c-wave (known as the early diastolic positive wave). The c-wave represents the dicrotic notch of the original PPG signal. The dicrotic notch is associated with the closure of the aortic valve and subsequent retrograde blood flow. In it was shown that the b/a ratio is associated with increased arterial stiffness: thus, the ratio of b over a increases with age.

[Conference proceedings: ... Annual International Conference of the IEEE Engineering in Medicine and Biology Society. IEEE Engineering in Medicine and Biology Society. Conference -= August 2016]

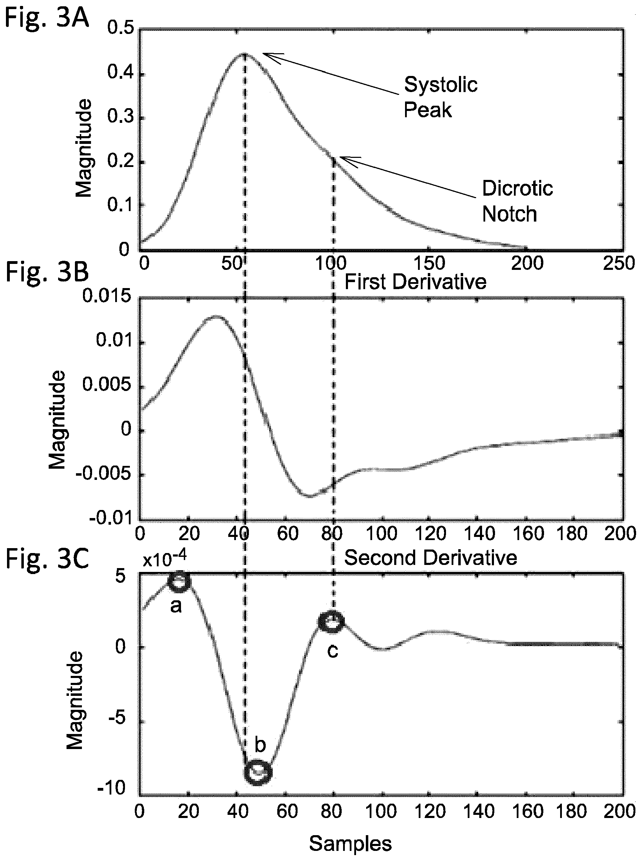

Attention is directed to **Fig. 3****,** which illustrates a PPG together with its first and second derivatives.

Accordingly, the initial PPG as well as the APG (and its component a-wave, b-wave, and c-wave) can be utilized in the generation of sound - as in example methods described below.

In some embodiments, the initial PPG as well as the APG (and its component a-wave, b-wave, and c-wave) can be further utilized in the generation of visualizations - as in example methods described below.

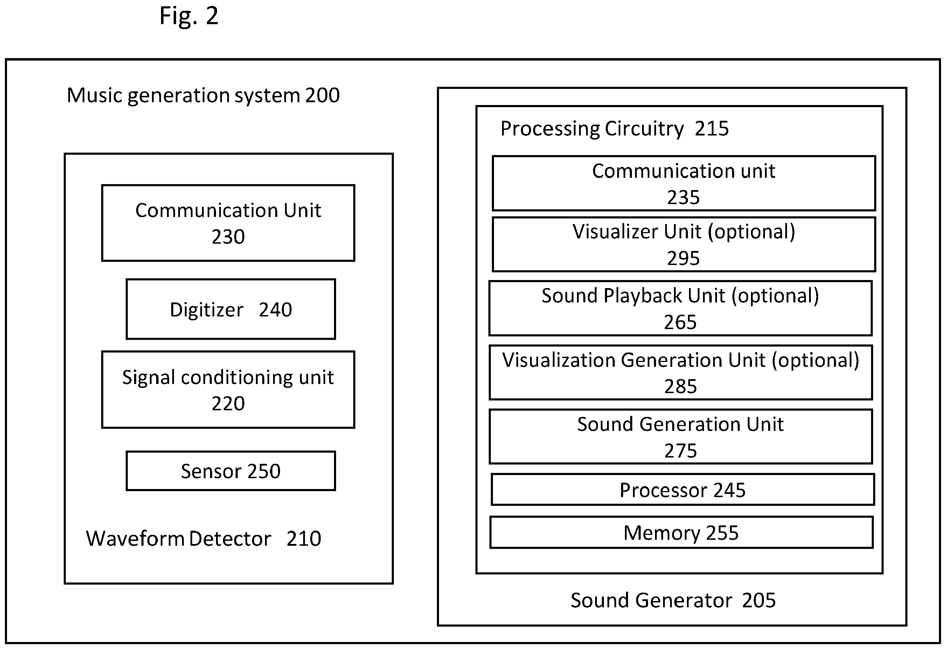

Attention is directed to **Fig. 2**, which illustrates a block diagram of an example music generation system, in accordance with some embodiments of the presently disclosed subject matter.

Music generation system **200** can include a waveform detector **210** and a sound generator **205**. In some embodiments waveform detector **210** is an integrated physical unit which communicates with sound generator **205** via a wireless connection such as Bluetooth. In some such embodiments, sound generator **205** can be partially or fully incorporated in a general purpose processing system such as a smartphone.

In some other embodiments, waveform detector **210** and sound generator **205** can communicate via a wired connection, be physically integrated into a single unit, or be operably connected in another suitable manner.

Waveform detector **210** can provide data indicative of a PG to sound generator **205**. Sound generator **205** can generate sound or music in accordance with the PG. Sound generator **205** can make the generated sound available via various mechanisms e.g. playing the sound on a speaker, storing it as digital audio, or storing it in another form e.g. as a musical score.

Waveform detector **210** can include a sensor **250**. Sensor **250** can be a device that measures data indicative of the waveform of a user's arterial pulse. By way of non-limiting example, sensor **250** can be an optical sensor (such as a pulse oximeter) which applies light (e.g. from an LED) to the user's skin (e.g. a user's finger) and measures changes in light absorption. In some embodiments, the sensor can measure 100 times per second. In some embodiments, sensor **250** can provide sensed light absorption measurements to signal conditioning unit **220**. In some embodiments, sensor **250** can provide signals and/or data indicative of sensed light absorption measurements to signal conditioning unit **220**. The light absorption measurements can be indicative of a volume of blood within tissue of the user.

In other embodiments, sensor **250** can be a pressure sensor, accelerometer or other suitable device producing data indicative of a plethysmogram.

Signal conditioning unit **220** can be a suitable type of device which e.g. receives signals and/or data indicative of periodic light absorption measurements (or pressure measurements or other measurements as appropriate) from sensor **250**. Signal conditioning unit **220** can be implemented by an analog circuit, a digital signal processor (DSP) or other suitable processor, or a combination thereof. Signal conditioning unit **220** can perform filtering and/or amplification and/or other preprocessing operations its input to prepare for analog/digital conversion,

Digitizer **240** can be operably connected to signal conditioning unit **220** and can receive signals and/or data indicative of periodic light absorption measurements (or pressure measurements or other measurements as appropriate) following the signal preprocessing. Digitizer **240** can be an analog-to-digital converter (ADC) or similar device that converts its input to a digital signal, thereby generating digital signal data that is indicative of a PG.

Communication unit **230** can be operably connected to digitizer **240.** Communication unit **230** can receive a digital signal indicative of a PG from digitizer **240** and transmit it to sound generator **205**. Communication unit **230** can utilize, for example, a wireless communication method such as Bluetooth, or some other wired or wireless communication technique. In some embodiments, communication unit **230** can be absent, and waveform detector **210** and sound generator **205** can be physically collocated.

Sound generator **205** can include processing circuitry **215.** In some embodiments, processing circuitry **210** includes a processor **245** and memory **255**.

Processor **245** can be a suitable hardware-based electronic device with data processing capabilities, such as, for example, a general purpose processor, digital signal processor (DSP), a specialized Application Specific Integrated Circuit (ASIC), one or more cores in a multicore processor etc. Processor **245** can also consist, for example, of multiple processors, multiple ASICs, virtual processors, combinations thereof etc.

Memory **135** can be, for example, a suitable kind of volatile and/or nonvolatile storage, and can include, for example, a single physical memory component or a plurality of physical memory components. Memory **135** can also include virtual memory. Memory **135** can be configured to, for example, store various data used in computation.

Processor **245** can be configured to execute several functional modules in accordance with computer-readable instructions implemented on a non-transitory computer-readable storage medium. Such functional modules are referred to hereinafter as comprised in the processor. These modules can include, for example, sound generation unit **275**, optional sound playback unit **265**, optional visualization generation unit **285,** optional visualizer unit **295** and communication unit **235.**

Communication unit **230** can receive data indicative of a PG from waveform detector **210** and provide it to sound generation unit **265**. Communication unit **230** can utilize, for example, a wireless communication method such as Bluetooth, or some other wired or wireless communication technique. In some embodiments, communication unit **230** can be absent, and waveform detector **210** and sound generator **205** can be physically collocated.

Sound generation unit **265** can receive data indicative of a PG (e.g. from communication unit **230**) can generate sound data from the PG. Example methods for deriving sound and music from the PG are described below.

In some embodiments, optional visualization generation unit **265** can receive data indicative of a PG (e.g. from communication unit **230**) and can generate visualizations from of the PG. Example methods for deriving visualizations from the PG are described below.

Optionally, sound playback unit **265** can receive generated sound data from sound generation unit **275** and play out the sound on e.g. a speaker or another sound reproduction or sound capture device. In some embodiments, sound playback unit **265** can store generated sound as digital audio or in another form e.g. as a musical score.

In some embodiments, sound playback unit **265** can include a number of oscillators (e.g. 3), each of which supports 1 or more waveforms (e.g. sine, square, triangle) and a number of frequencies (e.g. 12). In this case, the input to playback unit **265** can specify the frequency and waveform to be played by each oscillator for a particular time duration.

Optionally, visualizer unit **295** can receive visualization data from visualization generation unit **285** and display visualization on e.g. a screen, virtual reality (VR) helmet, or other display device. The visualizations can be video, a series of still images, a light sequence, or another form of visual experience. The displayed visualizations can be synchronized to sound playback of sound playback unit **265**.

In some embodiments, visualizer unit **295** can store generated visualization as a series of digital images, digital video or in another suitable form.

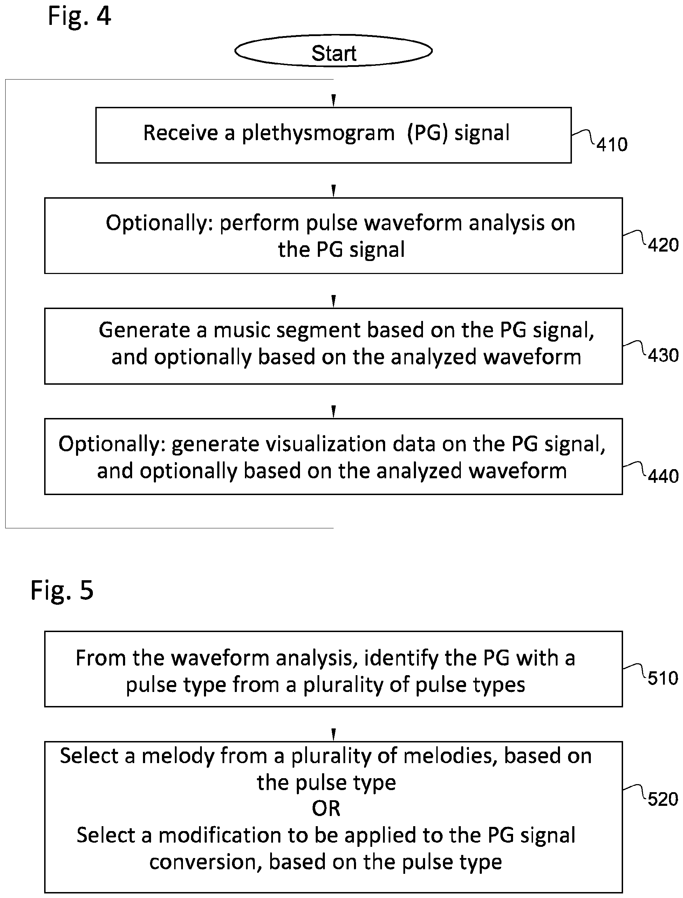

Attention is now drawn to **Fig. 4****,** which illustrates a flow diagram of an example method of deriving sound from a user's PG, in accordance with some embodiments of the presently disclosed subject matter.

The processing circuitry **215** (e.g. sound generation unit **275**) can receive (**410**) a PG signal e.g. a PG signal prepared by waveform detector **210** and received via communication unit **235**. The received PG signal can represent the user's arterial pulse over a particular interval of time e.g. 1 second.

The processing circuitry **215** (e.g. sound generation unit **275**) can optionally perform (**420**) waveform analysis on the PG signal e.g. compute the APG signal as described above.

The processing circuitry **215** (e.g. sound generation unit **275**) can generate a music segment (e.g. of 1 second duration) based on the PG signal.

By way of non-limiting example, the processing circuitry **215** (e.g. sound generation unit **275**) can perform the following:
a. determining, for a given time, an amplitude of the digital signal for that time;
b. selecting an audio tone based on the determined amplitude.

By way of non-limiting example: processing circuitry **215** (e.g. sound generation unit **275**) can map the newly determined amplitude to some number that represents an identifier of an audio tone from a maintained set of recorded audio tones (e.g. in some embodiments there may be e.g. twelve ranges of amplitude, and each of these ranges can then be associated with one of 12 tones).

Processing circuitry **215** (e.g. sound generation unit **275**) can then repeat steps a.-b. for zero or more additional iterations. By performing additional iterations, subsequent changes in the PG-based signal (and amplitude) will result in subsequent tones being selected - resulting in a sequence of tones derived from the PG.

Alternatively the processing circuitry **215** (e.g. sound generation unit **275**) can generate sound data based on the waveform analysis - instead of or in addition to the PG signal itself. Example methods for utilizing the results of waveform analysis in sound generation are described below with reference to **Fig. 5****.** The generated data can be in the format utilized by sound playback unit **265** and can then be played via sound playback unit **265**. In this case the tones that are selected can be combinations of frequency values and waveforms to be used by the 3 oscillators (e.g. each of 16 PG amplitude ranges can be mapped to a respective tuple, where each tuple includes 3 oscillator frequencies with associated wave forms).

Alternatively, the generated data can be written to a storage medium (e.g. a disk) in a particular format such as a digital audio format, or as a musical score etc.

Optionally. processing circuitry **215** (e.g. visualization generation unit **285**) can generate a visualization segment (e.g. an image, a sequence of still images of some duration, or a video segment of some duration e.g. 1 second) based on the PG signal. Optionally, the generation of visualizations can be based on the waveform analysis instead of or in addition to the PG signal itself.

The generated data can be in the format utilized by optional visualizer unit **295** and can then be displayed via optional visualizer unit **295**.

Alternatively, the generated data can be written to disk in a particular format such as a digital image format, digital video format etc.

The processing circuitry **215** (e.g. sound generation unit **275**) can then receive (410) the next PG signal.

Attention is directed to **Fig. 5****,** which illustrates a flow diagram of an example method of utilizing waveform analysis to generate or modify the music derived from the PG, in accordance with some embodiments of the presently disclosed subject matter.

In some embodiments, the processing circuitry **215** (e.g. sound generation unit **275**) can - utilizing the results of the waveform analysis -identify a particular PG pulse type from a plurality of pulse types. (e.g. in accordance with the presence and/or shape of the dichronic notch).

In some embodiments, the processing circuitry **215** (e.g. sound generation unit **275**) maintains a library of melodies (where each melody is a sequence of tones). The library can contain some number of melodies (e.g. 10, 100 or some other number). The melodies can have some duration e.g. 1 second.

Each of the melodies in the library can be associated with one or more PG pulse types. The PG pulse type associated with a melody can be in accordance with waveforms resulting from waveform analysis (e.g. a short-time Fourier transform, Gabor Transform, Wigner distribution function etc.) applied to the melody. More specifically: a melody can be associated with a particular PG waveform according to the similarity of its analyzed waveform to a PG pulse type.

The processing circuitry **215** (e.g. sound generation unit **275**) can then select (**520**) a melody from the library of melodies with a pulse type that matches the identified PG pulse type. The selected melody can then constitute part or all of the music segment.

In some embodiments, processing circuitry **215** (e.g. sound generation unit **275**) does not select a melody according to the pulse type, but rather selects the plurality of tones from which the sequence of tones is subsequently generated. For example: processing circuitry **215** (e.g. sound generation unit **275**) can maintain multiple pluralities of tones e.g. one plurality might consist of flute tones, while another plurality might consist of piano tones. Processing circuitry **215** (e.g. sound generation unit **275**) can then select the specific plurality corresponding to the identified pulse type, and then select individual tones of the plurality in response to amplitudes of the digital signal.

## Claims

1. A system of producing music that is in accordance with a user's measured arterial pulse wave form, the system comprising:
a sound generator comprising a processing circuitry, the processing circuitry comprising a processor and memory, the processing circuitry being configured to:
**a.** receive a digital signal indicative of a plethysmogram (PG) of the user; and
**b.** generate, from the digital signal, data indicative of an audio segment.

2. The system of claim 1, additionally comprising a waveform detector operably connected to the sound generator and configured to provide the digital signal to the sound generator, the waveform detector comprising:
**a.** a sensor configured to provide data indicative of a volume of blood within tissue of the user, and
**b.** a digitizer configured to:
generate, from the data indicative of the volume of blood, data indicative of a digital signal,
the digital signal being indicative of a PG.

3. The system of claim 1 or claim 2, wherein, to generate the data indicative of the audio segment, the processing circuitry is further configured to:
**a.** determine, for a given time, an amplitude of the digital signal;
**b.** select an audio tone from a plurality of audio tones in accordance with the determined amplitude; and
**c.** repeat a.-b. for zero or more additional times.

4. The system of any one of claim 1 or claim 2, wherein, to generate the data indicative of the audio segment, the processing circuitry is further configured to:
**a.** utilize waveform analysis to determine, for a given interval of the digital signal, a pulse type;
**b.** select a sequence of audio tones from a plurality of sequences of audio tones in accordance with the determined pulse type; and
**c.** repeat a.-b. for zero or more additional intervals.

5. The system of claim 1 or claim 2, wherein, to generate the data indicative of the audio segment, the processing circuitry is further configured to:
**a.** utilize waveform analysis to determine, for a given interval of the digital signal, a pulse type;
**b.** determine, for a given time, an amplitude of the digital signal;
**c.** select an audio tone from a first plurality of audio tones in accordance with the determined amplitude,
wherein the first plurality of audio tones is selected from a group of audio tones in accordance with the determined pulse type;
**d.** repeat b.-c. for zero or more additional times; and
e. repeat a.-d. for zero or more additional intervals.

6. The system of any one of claims 1-5, wherein the processing circuitry is further configured to play the audio segment on a speaker.

7. The system of any one of claims 1-6, wherein the processing circuitry is further configured to store data indicative of the audio segment to a storage medium.

8. The system of any one of claims 1-7, wherein the processing circuitry is further configured to store, to a storage medium, musical score data that is in accordance with the audio segment.

9. The system of any one of claims 1-8, wherein the processing circuitry is further configured to generate visualization data in accordance with the digital signal.

10. The system of claim 2, wherein the sensor is selected from a group consisting of:
a pulse oximeter,
a blood pressure sensor, and
an accelerometer.

11. A processing circuitry-based method of producing music that is in accordance with a user's measured arterial pulse wave form, the method comprising:
**a.** receiving a digital signal indicative of a plethysmogram (PG) of the user; and
**b.** generating, from the digital signal, data indicative of an audio segment.

12. A computer program product comprising a computer readable non-transitory storage medium containing program instructions, which program instructions when read by a processing circuitry, cause the processing circuitry to perform a method of producing music that is in accordance with a user's measured arterial pulse wave form, the method comprising:
**a.** receiving a digital signal indicative of a plethysmogram (PG) of the user; and
**b.** generating, from the digital signal, data indicative of an audio segment.
